# EUROPEAN PATENT SPECIFICATION

(11) **EP 0 409 134 B1**
(45) Date of publication and mention of the grant of the patent: **02.02.1994**
(21) Application number: 90113591.3
(22) Date of filing: 16.07.1990
(51) Int. Cl.: A61M 5/50

(54) **A non-reusable syringe**
Einmalspritze
Seringue à emploi unique

(30) Priority: 17.07.1989 ES 8902512
(43) Date of publication of application: 23.01.1991
(73) Proprietor: COMERCIAL MARIAE, S.L., E-31005 Pamplona (ES); Sempere Escudero, Philippe, E-08015 Barcelona (ES)
(72) Inventor: Sempere Escudero, Philippe, 08015 Barcelona (ES)
(74) Representative: Duran Moya, Luis-Alfonso

(56) References cited:
- WO-A-88/10127
- WO-A-89/02759
- WO-A-89/06146

## Description

This invention refers to a syringe offering a high degree of safety in that it possesses characteristics which ensure that it cannot be used a second time.

There have been numerous types of syringe recently made available. Most relevant example of the status of the art are to be found in WO-A- 8 810 127 and in GB-A- 2 015 833.

There is no particular difficulty in inventing a syringe which cannot be reutilized after the first application but the following factors require to be taken into consideration:
- Simple construction allowing ease of operation by the user.
- Low manufacturing cost, at least as low as the cost of existing syringes and even less as is the case with the syringe described in this patent.
- The syringe must be absolutely incapable of being reused after the first use, this requirement being primordial and being totally independent of the wishes of the user.
- The operation of the syringe must not depend on complicated components which not only increase the cost but increase the possibility of overiding the very objective of non-reutilization. The more sophisticated the mechanism, the easier it is to thwart the intention of the design. This implies a simple design incorporating the necessary impediments to reutilization.

To achieve these four important factors, the object is not just to avoid complicating the syringe or not, but primarily to combine simplicity of design with the above qualities.

According to the invention, a solution is proposed to reach the above scope, which is represented by a syringe having the characteristics defined in claim 1.

The accompanying drawings will assist in understanding the description of the non-resusable syringe object of this invention patent.

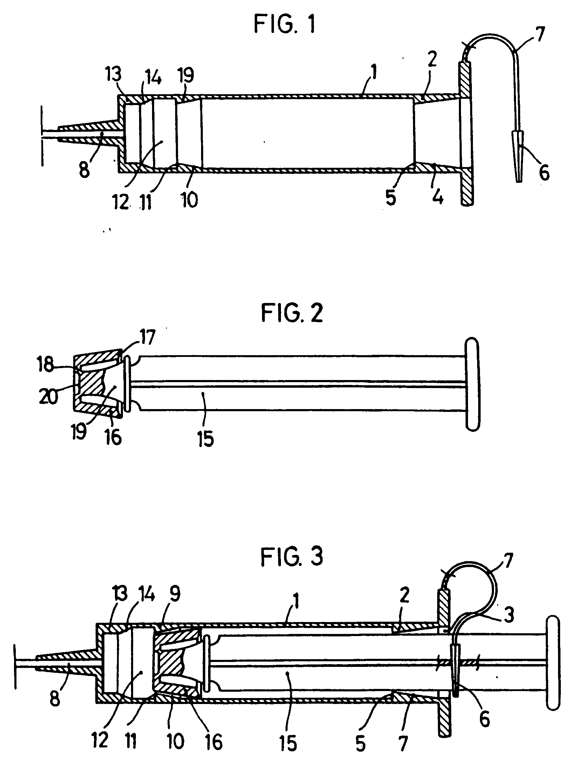

Figures 1 and 2 are respectively a longitudinal cross-section and an external view in part section of the cylinder and piston respectively.

Figure 3 is a longitudinal cross section of the syringe before use.

Figures 4 and 5 are detail sections showing the syringe during use and after use respectively.

Figure 6 is a section showing the working of one of the safety devices in the syringe which is the object of this patent.

Figure 7 is an enlarged detail of the syringe piston.

As is shown in the drawings the syringe, object of this patent is characterized by the interior of the cylindrical body -1- having a series of reliefs on the diameter which constitute the safety characteristics and comprise essentially an initial step -2- near the entry mouth -3- -of the syringe body, this step having a cone or entry ramp -4- and a zone -5- which is generally straight and perpendicular to the interior surface of the body -1-. This step is designed to prevent the extraction of the piston assembly which, in this syringe object of this present invention, is supplied fitted within the body -1- and retained by means of a transverse pin -6- attached to the body -1- by a tie component -7- or some other means designed to prevent displacement of the piston to the outlet position before its required operation.

Another step -9- is provided near the outlet -8- extremity of the syringe body, this also having a cone or ramp -10- and a straight zone -11-, following which there is a safety area -12- and a further narrower section provided by increased wall thickness -13- near the extremity with its cone or entry ramp -14-.

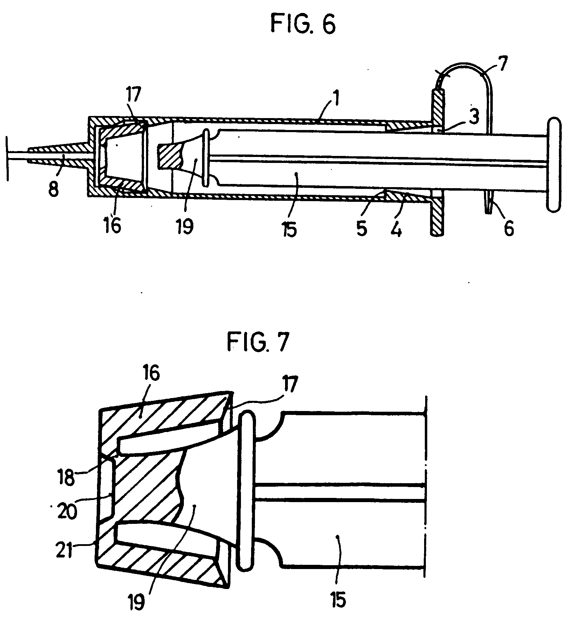

Corresponding to the cross sectional variations described above the piston comprises a body or plunger stem -15- which has attached to its internal extremity an expansible hood -16- as is shown in the detail figure 7. This drawings shows that the hood -16- is conical with a flat or slightly conical rear face -17-. The hood -16- is expansible by virtue of its own elasticity acting as the piston head adjusting itself to the interior surface of the body -1- of the syringe. The hood is joined to the stem -15- of the piston by means of a section -18- having a smaller thickness uniting it to the extremity -19- which is a prolongation of the stem -15- of the piston. This smaller thickness section -18- which is designed accordingly is obtained by forming a cavity -20- in the front face -21- of the said hood -16-.

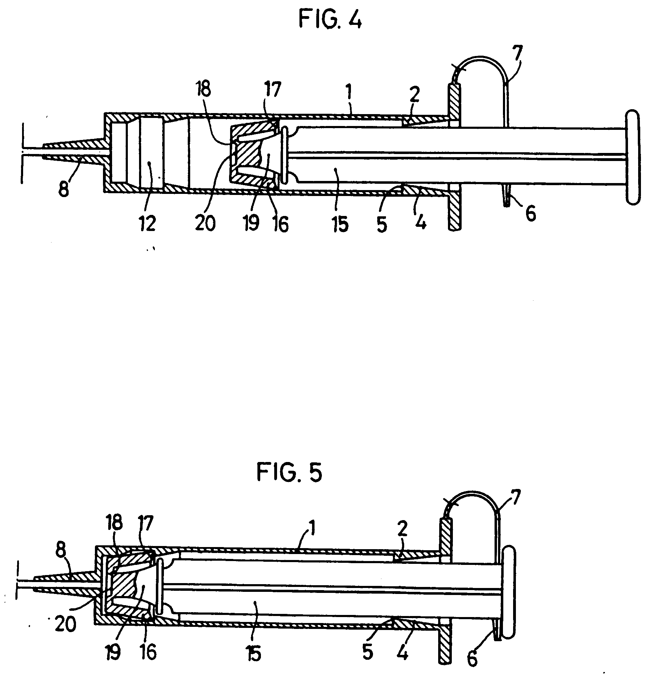

By means of the above described configuration a situation is obtained in which once the plunger has has been driven to the bottom, as shown in figure 5, to expel the liquid, the piston cannot be extracted for a second utilization of the syringe bein so prevented by the obstacle presented by the step -9- against the edge of the hood -17-. Should a persistent effort be made to extract the piston the thin sectioned zone -18- will break as is shown in figure 6, the syringe being then unusable.

Some of the most important aspects of this novel syringe designed for one only operation are summarized below.

### MANUFACTURE:

A most important feature of the novel syringe is that it comprises only two mouldings. In this type of product necessarily produced in very considerable quantities a differnce in mould costs of fractions of a cent will amount to vast sums in total.

### APPEARANCE:

The outward appearance of this syringe is not dissimilar to existing syringes and this simularity is considered by the inventor to be important so as not deter the user who can readily accept minor variations without varying his practices unduly.

### SAFETY ZONES:

The fact that the plunger, before use, is withdrawn a few millimetres is precisely the element that provides the four qualities mentioned previously.The user appreciative of its characteristic, in his own interests will soon adjust himself to this operation prior to its utilization.

For others not concerned about the multiple use of a syringe, despite their reticence in this respect, there is no alternative method of use with this novel syringe with its ineludible safety feature.

### THE PLUNGER

Its conicity allows it to adapt perfectly to the interior of the cylinder. The controlled dimension of the cone ensures a correct expulsion or withdrawal of liquid while the adequate flexibility allows it to negotiate the ramp sections smoothly though having sufficient resistance to remain blocked in its passage by the salients provided.

The reduced dimensioned section of the cone, made to precise fragility ensures its breakage in the event of an attempt to force it against the blockage positions in pulling or pushing.

### TOTAL SECURITY:

Overiding the safety characteristics of this novel syringe is impossible, any attempt so to do making it unusable. The most delicate component is the plunger, as well as being the most important and hence it is provided with four safety stages.

FIRST STAGE: As in other syringes, this one is supplied with the piston already housed in the cylinder. At one centimetre from the base of the cylinder internally there is a salient of such a form that in the course of assembly the piston may enter only once in the cylinder but cannot there after be extracted without breaking.

Nevertheless if by some effort the piston succeeds in being withdrawn and the cone is reduced on its circumference, forming the piston head, so that it is no longer stopped by the salients at the extremity of the cylinder, the head will no longer function having been designed precisely to suit the cylinder dimension for extracting or injecting liquid.

SECOND STAGE: Should there be an attempt to withdraw the piston and the head from the safety zone the cone will disconnect from the stem, and the broken plunger will be completely unusable.

THIRD STAGE: Should the piston be pushed, instead of being drawn, by exerting pressure through the liquid outlet orifice, with a view to recuperating it intact, breakage will occur immediately as in the previous cases.

FOURTH STAGE: This last and important stage is explained in the paragraph entitled: Reutilization impossible: below.

### THE RAMPS AND SALIENTS.

The design both of the ramps -4- and -10- as well as the stop or entry ramp -14- is such that the piston movement is smooth and is able to pass without hesitation, the salients provided for its blockage.

The required progressive light pressure applied to the piston offers no difficulty in the operation of painless and correct injection.
THE SAFETY PIN: This pin or key on the outside of the cylinder fulfils two functions:

FIRST: Prevents the plunger becoming blocked and hence useless between the time of manufacture and its required use.

SECOND: It is advisable not to remove this pin when extracting blood for obvious reasons as with any other syringe.

### REUTILIZATION IMPOSSIBLE:

A user trying to use this syringe a second time will meet with major obstacles rendering the reutilization almost impossible.

Having extracted the substance to be injected, the air contained in the safety zone remains and he will try to inject solely the substance assuming that the piston having reached the end of the travel he would have used this syringe like any other on the market.

This category of user will immediately realize that not having purged his syringe, and by the inclination he has to give it, particularly for intravenous injections, not only is he injecting air that was not extracted but also that tee safety zone will automatically fill with the substance contained in the syringe.

It is evident that in the incomprehensible misjudgement of someone using this syringe on a second occasion he will be incurring the risk of injecting air in the vein and depreciate small quantity of his precious and costly substance, this being the only way that the syringe can be re-used, but for this type of user there is no way in which this syringe can be used several times, however much he may try.

### FOURTH STAGE:

Taking into consideration the type of materials specifically approved for this type of application and considering both their characteristics and the mode of application the inventor is satisfied that the piston is incapable of performing functions beyond those required for carrying out one injection correctly.

The more one moves the plunger up and down the less its efficiency, the cone closing in until it is no longer capable of impelling or extracting liquid.

The material being very ductile it is possible to re-form it so that it continues to function as a piston but to achieve this it has to be withdrawn from the cylinder, which is impossible without breaking it.

However the hypothetical and cojectural possibility of reutilization carries with it risks which would discourage such a temptation, hardly worthwhile and with very limited results.

As far as the normal professional user is concerned, it is difficult to imagine that such a person would disregard the dangers of injecting air into the patient or the inefficacity of injecting less than the prescribed dosage.
ANOTHER CHARACTERISTIC of this novel syringe is that it can be manufactured in all the sizes and types required by the various uses for which it may be destined.

### BRIEFLY:

Given the basic qualities which characterize this invention it is easy to understand that the user will accustom himself to the 0,50 cm. blockage space, which could however well be of another dimension; there being hence no specific explanation for the use of this syringe, its functioning being no different to other existing types.

The patent offers consequently a novel syringe with full safety characteristics ensuring its non utilization, in the opinion of the author contributing a major social benefit and a solution to the problem of multi-usage of syringes with their dangers to everyone.

## Claims

1. A non-reusable syringe, comprising a cylindrical body (1), a piston assembly arranged moveable inside the cylindrical body (1) which is formed by a piston stem (15) and a piston head (16) connected with the piston stem (15) in a breakable manner, whereby the cylindrical body (1) has a first relief near the bottom end of the syringe and a second relief near the top end of the syringe to engage with the piston head (16), thereby preventing complete extraction of the piston assembly and reuse of the syringe, characterized in that the piston head comprises a conic reversed hood (16), which is expansible and capable of maintaining a sealing contact against the cylindrical body (1), and in that the breakable connection of said hood with the stem (15) consists in a dimensionally controlled thin section (18) formed in the top wall of the hood.

2. A non-reusable syringe as in claim 1, characterized in that the thickness of said breakable section (18) is determined by the depth of a cavity formed in the front face of the hood.

## Patentansprüche

1. Einwegspritze, die einen zylindrischen Körper (1) und einen Kolbenaufbau umfaßt, der innerhalb des zylindrischen Körpers (1) beweglich angeordnet ist, der von einer Kolbenstange (15) und einem Kolbenkopf (16) gebildet wird, der in abbrechbarer Weise mit der Kolbenstange (15) verbunden ist, wobei der zylindrische Körper (1) in der Nähe des unteren Endes der Spritze eine erste Aussparung und in der Nähe des oberen Endes der Spritze eine zweite Aussparung aufweist, die in den Kolbenkopf (16) eingreifen, wodurch das vollständige Herausziehen des Kolbenaufbaues und die Wiederverwendung der Spritze vermieden werden, dadurch gekennzeichnet,
daß der Kolbenkopf (16) eine konische Form aufweist und auf Grund seiner eigenen Elastizität dehnbar ist und auf seiner Vorderseite (21) eine vorgegebene Bruchstelle (18) aufweist.

2. Einwegspritze nach Anspruch 1, durch das Verfahren gekennzeichnet, bei dem die Dicke des Sicherheitsteiles durch einen Hohlraum bestimmt wird, der in der Vorderseite der Kappe gebildet wird, wodurch eine Zone für einen geregelten Bruch gebildet wird.

## Revendications

1. Seringue à jeter, comprenant un corps cylindrique (1), un ensemble piston disposé de façon à pouvoir se déplacer à l'intérieur du corps cylindrique (1) et formé par une tige de piston (15) et une tête de piston (16) raccordée à la tige de piston (15) de façon à pouvoir être séparée par cassure, le corps cylindrique (1) présentant une première partie en saillie à proximité de l'extrémité arrière de la seringue et une seconde partie en saillie à proximité de l'extrémité avant de la seringue pour s'emboîter avec la tête de piston (16), évitant ainsi l'extraction complète de l'ensemble piston et la réutilisation de la seringue, caractérisée en ce que la tête de piston comprend un capuchon conique inversé (16) expansible et capable de se maintenir en contact hermétique contre le corps cylindrique (1) et en ce que le raccord à casser dudit capuchon à la tige (15) consiste en une portion mince à dimensions contrôlées (18) formée dans la paroi supérieure du capuchon.

2. Seringue à jeter selon la revendication 1, caractérisée en ce que l'épaisseur de ladite portion (18) à casser est déterminée par la profondeur d'une cavité formée dans la face frontale du capuchon.
